# EUROPEAN PATENT APPLICATION

(11) **EP 4 339 521 A1**
(43) Date of publication of application: **20.03.2024**
(21) Application number: 22816155.0
(22) Date of filing: 01.06.2022
(51) Int. Cl.: F24F 6/00, A61L 2/10, F24F 8/22

(54) **HUMIDIFIER**

(30) Priority: 03.06.2021 JP 2021093737
(71) Applicant: Daikin Industries, Ltd., Osaka-shi, Osaka 530-0001 (JP)
(72) Inventor: KOYAMA, Chika, Osaka-shi, Osaka 530-0001 (JP); SAITO, Tomoki, Osaka-shi, Osaka 530-0001 (JP); TANAKA, Toshio, Osaka-shi, Osaka 530-0001 (JP); OKUMOTO, Mamoru, Osaka-shi, Osaka 530-0001 (JP); KUROI, Kiyoshi, Osaka-shi, Osaka 530-0001 (JP)
(74) Representative: Goddar, Heinz J.
(86) International application number: PCT/JP2022/022334
(87) International publication number: WO 2022/255410

(57) **Abstract**

A housing (20) is formed with an air passage (23) through which air passes and a water storage tank (24) configured to store water. A humidifying rotor (30) extends over the air passage (23) and the water storage tank (24), and is configured to rotate so that a portion of the humidifying rotor (30) having absorbed moisture from the water storage tank (24) moves to the air passage (23) and releases the moisture. The irradiator (40) irradiates part of the humidifying rotor (30) with ultraviolet light.

## Description

### TECHNICAL FIELD

The present disclosure relates to a humidifier.

### BACKGROUND ART

Patent Document 1 discloses a humidifier including a water tank configured to hold water, a vaporization filter configured to absorb the water from the water tank and vaporize the water, a photocatalyst attached to the surface of the vaporization filter, and an ultraviolet lamp configured to irradiate the vaporization filter with ultraviolet light.

### CITATION LIST

### PATENT DOCUMENT

Patent Document 1: Japanese Unexamined Patent Publication No. 2006-3042

### SUMMARY OF THE INVENTION

### TECHNICAL PROBLEM

In the humidifier of Patent Document 1, it is necessary to increase the output of the ultraviolet lamp in order to irradiate a wide area of the vaporization filter with ultraviolet light. For this reason, it is difficult to reduce the output of the ultraviolet lamp.

### SOLUTION TO THE PROBLEM

A first aspect of the present disclosure is directed to a humidifier. The humidifier includes a housing (20) formed with an air passage (23) through which air passes and a water storage tank (24) configured to store water; a humidifying rotor (30) extending over the air passage (23) and the water storage tank (24) and configured to rotate so that a portion of the humidifying rotor (30) having absorbed moisture from the water storage tank (24) moves to the air passage (23) and releases the moisture; and an irradiator (40) configured to irradiate part of the humidifying rotor (30) with ultraviolet light.

In the first aspect, the humidifying rotor (30) is irradiated with ultraviolet light, and thus microorganisms adhering to the humidifying rotor (30) can be sterilized or inactivated. Thus, microbial growth in the humidifying rotor (30) can be reduced.

In addition, in the first aspect, part of the humidifying rotor (30) can be irradiated with ultraviolet light while the humidifying rotor (30) is rotated. With this configuration, a wide area of the humidifying rotor (30) can be irradiated with ultraviolet light. Thus, the output of the irradiator (40) can be reduced in comparison with the case where a wide area of the humidifying rotor (30) is irradiated with ultraviolet light without the humidifying rotor (30) being rotated.

A second aspect of the present disclosure is an embodiment of the first aspect. The humidifier of the second aspect further includes a controller (73) configured to perform first control. In the first control, the controller (73) controls the humidifying rotor (30) and the irradiator (40) so that the irradiator (40) emits ultraviolet light when the humidifying rotor (30) is rotated.

In the second aspect, by performing the first control, part of the humidifying rotor (30) can be irradiated with ultraviolet light while the humidifying rotor (30) is rotated. With this configuration, a wide area of the humidifying rotor (30) can be irradiated with ultraviolet light. Thus, the output of the irradiator (40) can be reduced in comparison with the case where a wide area of the humidifying rotor (30) is irradiated with ultraviolet light without the humidifying rotor (30) being rotated.

A third aspect of the present disclosure is an embodiment of the second aspect. In the third aspect, if the first control is started when humidifying operation where the humidifying rotor (30) is rotated is in progress, the controller (73) controls the humidifying rotor (30) and the irradiator (40) so that the humidifying rotor (30) keeps rotating and the irradiator (40) starts emitting ultraviolet light.

In the third aspect, the first control can be performed when the humidifying operation is in progress.

A fourth aspect of the of the present disclosure is an embodiment of the second or third aspect. In the fourth aspect, if the first control is started when the humidifying operation where the humidifying rotor (30) is rotated is not in progress, the controller (73) controls the humidifying rotor (30) and the irradiator (40) so that the humidifying rotor (30) starts rotating and the irradiator (40) starts emitting ultraviolet light.

In the fourth aspect, the first control can be performed when the humidifying operation is not in progress.

A fifth aspect of the present disclosure is an embodiment of the fourth aspect. In the fifth aspect, the number of rotations of the humidifying rotor (30) in the first control performed when the humidifying operation is not in progress is less than the number of rotations of the humidifying rotor (30) in the first control performed when the humidifying operation is in progress.

In the fifth aspect, when the humidifying operation is not in progress, the amount of ultraviolet irradiation (product of the illuminance and the irradiation time) of the humidifying rotor (30) during one rotation of the humidifying rotor (30) can be increased. Thus, when the humidifying operation is not in progress, it is possible to improve a sterilization effect (effect of sterilizing the humidifying rotor (30) with ultraviolet light) during one rotation of the humidifying rotor (30).

A sixth aspect of the present disclosure is an embodiment of any one of the second to fifth aspects. In the sixth aspect, the controller (73) performs the first control every time a predetermined time elapses.

In the sixth aspect, the first control can be automatically performed every predetermined time. Thus, the first control can be periodically performed.

A seventh aspect of the present disclosure is an embodiment of the sixth aspect. In the seventh aspect, the predetermined time is settable.

In the seventh aspect, the cycle of the first control can be changed.

An eighth aspect of the present disclosure is an embodiment of any one of the second to seventh aspects. In the eighth aspect, the controller (73) controls timing of executing the first control according to an environmental condition around the humidifier.

In the eighth aspect, the timing of executing the first control can be controlled according to the environmental condition around the humidifier, which is correlated with the ease of microbial growth in the humidifying rotor (30). Thus, microbial growth in the humidifying rotor (30) can be reduced properly.

A ninth aspect of the present disclosure is an embodiment of any one of the second to eighth aspects. In the ninth aspect, the controller (73) controls the illuminance or irradiation time of ultraviolet light from the irradiator (40) in the first control according to the environmental condition around the humidifier.

In the ninth aspect, the amount of ultraviolet irradiation by the irradiator (40) (product of the illuminance and the irradiation time) in the first control can be controlled according to the environmental condition around the humidifier, which is correlated with the ease of microbial growth in the humidifying rotor (30). Thus, microbial growth in the humidifying rotor (30) can be reduced properly.

A tenth aspect of the present disclosure is an embodiment of any one of the second to ninth aspects. In the tenth aspect, the number of rotations of the humidifying rotor (30) is variable, and in the first control, the controller (73) controls the illuminance of ultraviolet light from the irradiator (40) according to the number of rotations of the humidifying rotor (30).

In the tenth aspect, it is possible to properly control the amount of ultraviolet irradiation (product of the illuminance and the irradiation time) of the humidifying rotor (30).

An eleventh aspect of the present disclosure is an embodiment of the tenth aspect. In the eleventh aspect, the controller (73) controls the number of rotations of the humidifying rotor (30) according to a humidification load in the humidifying operation where the humidifying rotor (30) is rotated.

In the eleventh aspect, the humidifying rotor (30) can promote humidification of air in the air passage (23) according to the humidification load, and thus humidification can be properly performed.

### BRIEF DESCRIPTION OF THE DRAWINGS

FIG. 1 is a schematic view illustrating an example configuration of a humidifier.
FIG. 2 is a block diagram illustrating the example configuration of the humidifier.
FIG. 3 is a schematic view illustrating an example of the area of ultraviolet irradiation by an irradiator.
FIG. 4 is a flowchart for describing first control.

### DESCRIPTION OF EMBODIMENTS

Embodiments will now be described in detail with reference to the drawings. Note that the same reference characters denote the same or equivalent components in the drawings, and the description thereof will not be repeated.

### (Embodiment)

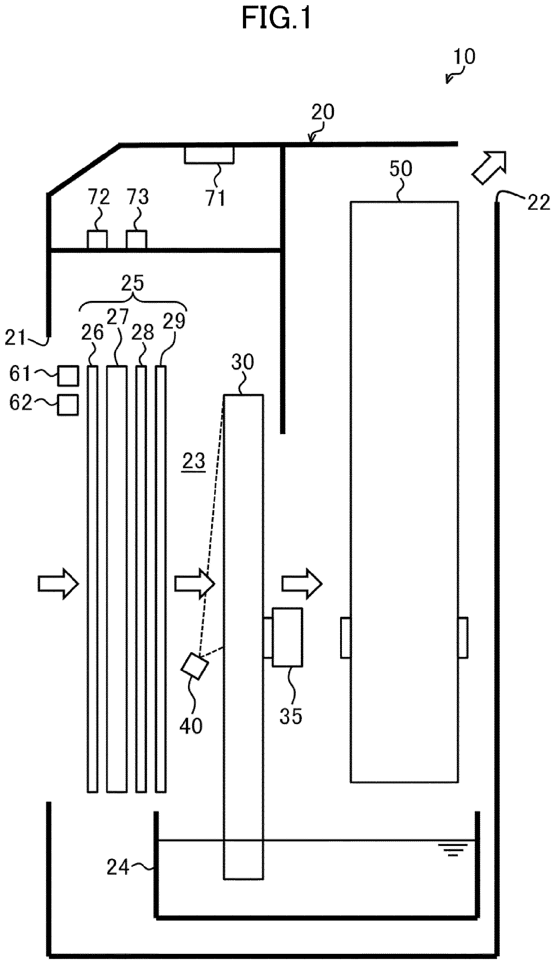

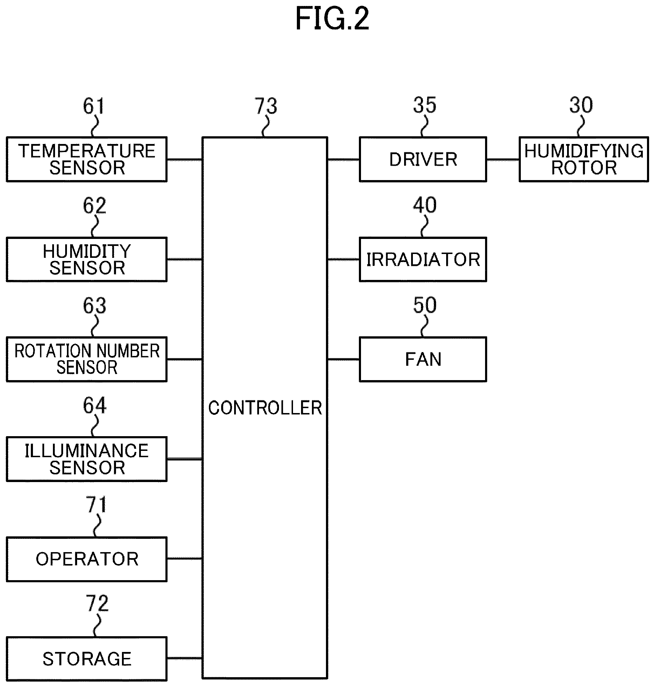

FIGS. 1 and 2 illustrate the configuration of a humidifier (10) of an embodiment. In this example, the humidifier (10) forms an air purifier having a humidifying function and an air purifying function. The humidifier (10) includes a housing (20), an air purification unit (25), a humidifying rotor (30), a driver (35), an irradiator (40), and a fan (50).

Hereinafter, for convenience of explanation, "the side of the humidifier (10) where a suction port (21) is formed" will be referred to as a "front side." The suction port (21) will be described in detail later. Terms "front," "back," "upper," and "lower" used in the following description correspond to the directions of the humidifier (10) placed on a horizontal surface.

### [Housing]

The housing (20) houses the components of the humidifier (10). In this example, the housing (20) houses the air purification unit (25), the humidifying rotor (30), the driver (35), the irradiator (40), and the fan (50).

The housing (20) has the suction port (21) and a blow-out port (22). The suction port (21) is an opening through which air outside the housing (20) is sucked into the housing (20). The blow-out port (22) is an opening through which air inside the housing (20) is blown out of the housing (20).

An air passage (23) and a water storage tank (24) are formed inside the housing (20). The air passage (23) is a passage through which air passes, and causes the suction port (21) and the blow-out port (22) to communicate with each other. The water storage tank (24) stores water.

In this example, the housing (20) is formed in a rectangular parallelepiped box shape which is flat in the front-to-back direction. The suction port (21) is formed in a front portion of the housing (20), and opens forward. The blow-out port (22) is formed in a back portion of the housing (20), and opens diagonally backward and upward.

In this example, in the air passage (23), air flows from the suction port (21) side toward the blow-out port (22) side. In the air passage (23), the air purification unit (25), the humidifying rotor (30), and the fan (50) are arranged in this order from the upstream side to the downstream side in an air flow direction. The water storage tank (24) is arranged below the air passage (23).

### <Air Purification Unit>

The air purification unit (25) purifies air flowing through the air passage (23). In this example, the air purification unit (25) includes a prefilter (26), an ionizer (27), an electrostatic filter (28), and a deodorizing filter (29). In this example, the prefilter (26), the ionizer (27), the electrostatic filter (28), and the deodorizing filter (29) are arranged in this order from the upstream side to the downstream side in the air flow direction.

The prefilter (26) traps relatively large dust particles in air sucked from the suction port (21) side in the air passage (23).

The ionizer (27) charges relatively small dust particles in air having passed through the prefilter (26). For example, the ionizer (27) has a linear electrode and a plate electrode facing each other, and applies voltage to both the electrodes to cause corona discharge between these electrodes. By such corona discharge, dust particles in air are charged to a predetermined charge (positively or negatively).

The electrostatic filter (28) electrically attracts and traps the dust particles charged by the ionizer (27).

The deodorizing filter (29) deodorizes air having passed through the electrostatic filter (28). For example, the deodorizing filter (29) has a honeycomb base material and a deodorant supported on the surface of the base material. Examples of the deodorant include an adsorbent for adsorbing a component to be treated, such as an odor substance or a harmful substance, and a catalyst for oxidatively decomposing the component to be treated.

### [Humidifying Rotor]

The humidifying rotor (30) has water absorbency, and is rotatable. The humidifying rotor (30) extends over the air passage (23) and the water storage tank (24). The humidifying rotor (30) rotates so that a portion of the humidifying rotor (30) having adsorbed moisture from the water storage tank (24) moves to the air passage (23) and releases the moisture.

Specifically, the humidifying rotor (30) has a portion positioned in the air passage (23) and a portion positioned in the water storage tank (24). The portion of the humidifying rotor (30) positioned in the water storage tank (24) absorbs water from the water storage tank (24). When the humidifying rotor (30) rotates, the portion of the humidifying rotor (30) having absorbed moisture from the water storage tank (24) moves to the air passage (23). The portion of the humidifying rotor (30) positioned in the air passage (23) releases the moisture to air in the air passage (23). Accordingly, the air in the air passage (23) is humidified. In addition, when the humidifying rotor (30) rotates, the portion positioned in the air passage (23) moves to the water storage tank (24).

In this example, the humidifying rotor (30) is formed in a disc shape. The humidifying rotor (30) is rotatably supported so that the center axis thereof coincides with a rotation axis (Q). The center axis (rotation axis (Q)) of the humidifying rotor (30) extends in the front-to-back direction along the air flow direction in the air passage (23). The lower portion of the humidifying rotor (30) is immersed in the water storage tank (24).

### [Driver]

The driver (35) rotationally drives the humidifying rotor (30). By controlling the driver (35), rotation of the humidifying rotor (30) can be controlled. For example, the driver (35) includes a power source such as a motor, and a power transmission mechanism such as a gear.

### [Irradiator]

The irradiator (40) irradiates part of the humidifying rotor (30) with ultraviolet light. Specifically, the irradiator (40) includes a light source that emits ultraviolet light. Examples of the light source include a light emitting diode (LED), a laser diode, an excimer lamp, and a mercury lamp. The wavelength of ultraviolet light emitted from the irradiator (40) is, for example, 180 nm or more and 350 nm or less, and preferably 220 nm or more and 300 nm or less.

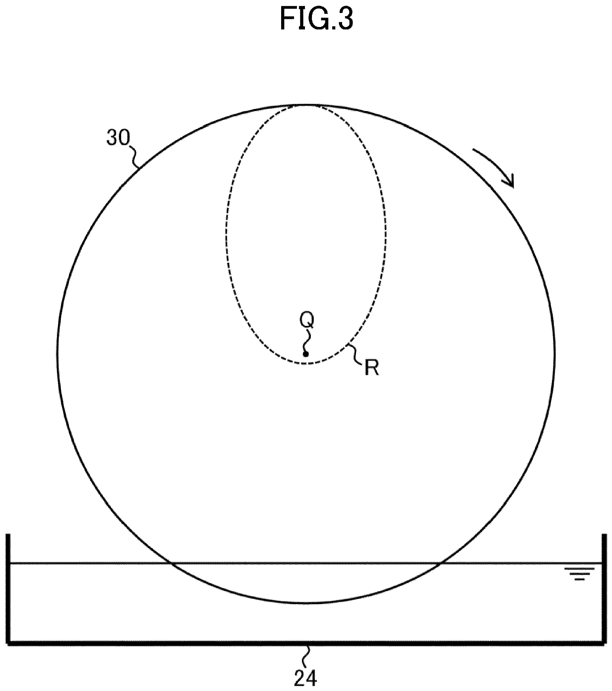

In this example, as illustrated in FIGS. 1 and 3, the irradiator (40) irradiates part of the portion of the humidifying rotor (30) positioned in the air passage (23) with ultraviolet light. The irradiator (40) emits ultraviolet light from the upstream side of the humidifying rotor (30) toward the end surface (end surface on the suction port (21) side) of the humidifying rotor (30) in the air flow direction in the air passage (23).

The irradiation area (R) of ultraviolet irradiation by the irradiator (40) is set so that the entire area of the humidifying rotor (30) is irradiated with ultraviolet light by one rotation of the humidifying rotor (30). In the example of FIG. 3, the irradiation area (R) includes the rotation axis (Q) of the humidifying rotor (30). The length of the irradiation area (R) in the radial direction (length in a direction perpendicular to the rotation axis (Q)) is longer than the radius of the humidifying rotor (30).

### [Fan]

The fan (50) generates the flow of air in the air passage (23). In this example, the fan (50) generates the flow of air from the suction port (21) to the blow-out port (22) in the air passage (23). For example, the fan (50) includes an impeller and a driver that rotationally drives the impeller. Examples of the fan (50) include a sirocco fan.

### [Sensor]

The humidifier (10) further includes various sensors. Detection results of these various sensors are transmitted to a controller (73) described later. In this example, the humidifier (10) includes a temperature sensor (61), a humidity sensor (62), a rotation number sensor (63), and an illuminance sensor (64). In FIG. 1, the rotation number sensor (63) and the illuminance sensor (64) are not shown.

The temperature sensor (61) detects the temperature of air around the humidifier (10). For example, the temperature sensor (61) is provided at the suction port (21) of the housing (20), and detects the temperature of air sucked into the housing (20) through the suction port (21) as "the temperature of air around the humidifier (10)." The temperature of air around the humidifier (10) is an example of an environmental condition around the humidifier (10).

The humidity sensor (62) detects the humidity of air around the humidifier (10). For example, the humidity sensor (62) is provided at the suction port (21) of the housing (20), and detects the humidity of air sucked into the housing (20) through the suction port (21) as "the humidity of air around the humidifier (10)." The humidity of air around the humidifier (10) is an example of the environmental condition around the humidifier (10).

The rotation number sensor (63) detects the number of rotations of the humidifying rotor (30).

The illuminance sensor (64) detects the illuminance of ultraviolet light emitted from the irradiator (40).

### [Operator]

The humidifier (10) further includes an operator (71). The operator (71) is given operation by an operating person of the humidifier (10). The operator (71) outputs a signal corresponding to the given operation. With such a configuration, the operating person can input information and instructions to the humidifier (10) by operating the operator (71). Examples of the operator (71) include an operation button provided on the housing (20), an operation panel, and a remote controller (not shown) of the humidifier (10).

### [Storage]

The humidifier (10) further includes a storage (72). The storage (72) is housed in the housing (20). The storage (72) stores various types of information. For example, the storage (72) stores information to be used to control each unit of the humidifier (10). Examples of the information to be used to control each unit of the humidifier (10) include values detected by the various sensors, set values used for various types of control, and various measured times.

### [Controller]

The humidifier (10) further includes the controller (73). The controller (73) is connected to each unit of the humidifier (10) via communication lines. In this example, the controller (73) is connected to the driver (35) that rotationally drives the humidifying rotor (30), the irradiator (40), the fan (50), the temperature sensor (61), the humidity sensor (62), the rotation number sensor (63), the illuminance sensor (64), the operator (71), and the storage (72). For example, the controller (73) controls rotation of the humidifying rotor (30) by controlling the driver (35).

The controller (73) controls each unit of the humidifier (10) according to the detection results of the various sensors so that various types of operation and control are performed in the humidifier (10). For example, the controller (73) has a processor and a memory that stores programs and information for operating the processor. When the processor executes the programs, various functions of the controller (73) are implemented.

In this example, the humidifier (10) performs humidifying operation. The controller (73) performs first control.

### [Humidifying Operation]

Next, the humidifying operation will be described. The humidifying operation is operation for humidifying air in the air passage (23) by rotating the humidifying rotor (30). For example, when the operator (71) is given operation for starting the humidifying operation, the controller (73) controls, in response to that operation, each unit of the humidifier (10) to perform the humidifying operation.

In the humidifying operation, when the driver (35) is in the driving mode, the humidifying rotor (30) is in the driving mode. The fan (50) is in the driving mode. The ionizer (27) of the air purification unit (25) is in the driving mode (where, for example, voltage is applied to the linear electrode and the plate electrode). As described above, when the humidifying operation is in progress, the humidifying rotor (30), the fan (50), and the ionizer (27) are in the driving mode.

When the fan (50) is driven, air outside the housing (20) is sucked into the air passage (23) through the suction port (21). The air sucked into the air passage (23) is purified by passing through the air purification unit (25), and thereafter, passes through the humidifying rotor (30).

When the humidifying rotor (30) is driven, the portion of the humidifying rotor (30) having absorbed moisture from the water storage tank (24) moves to the air passage (23). The air passing through the humidifying rotor (30) in the air passage (23) is humidified by the moisture being released from the portion of the humidifying rotor (30) positioned in the air passage (23).

The air having passed through the humidifying rotor (30) in the air passage (23) is sucked into the fan (50), and is blown out of the housing (20) through the blow-out port (22).

When the humidifying operation is not performed, the driver (35) is in the stop mode, and thus the humidifying rotor (30) is in the stop mode. The fan (50) and the ionizer (27) are also in the stop mode.

### [First Control]

The first control is control for irradiating the humidifying rotor (30) with ultraviolet light to reduce microbial growth in the humidifying rotor (30). Examples of microorganisms include molds and bacteria. Here, the microorganisms may include viruses. In the first control, the controller (73) controls the humidifying rotor (30) and the irradiator (40) so that the irradiator (40) emits ultraviolet light when the humidifying rotor (30) is rotated.

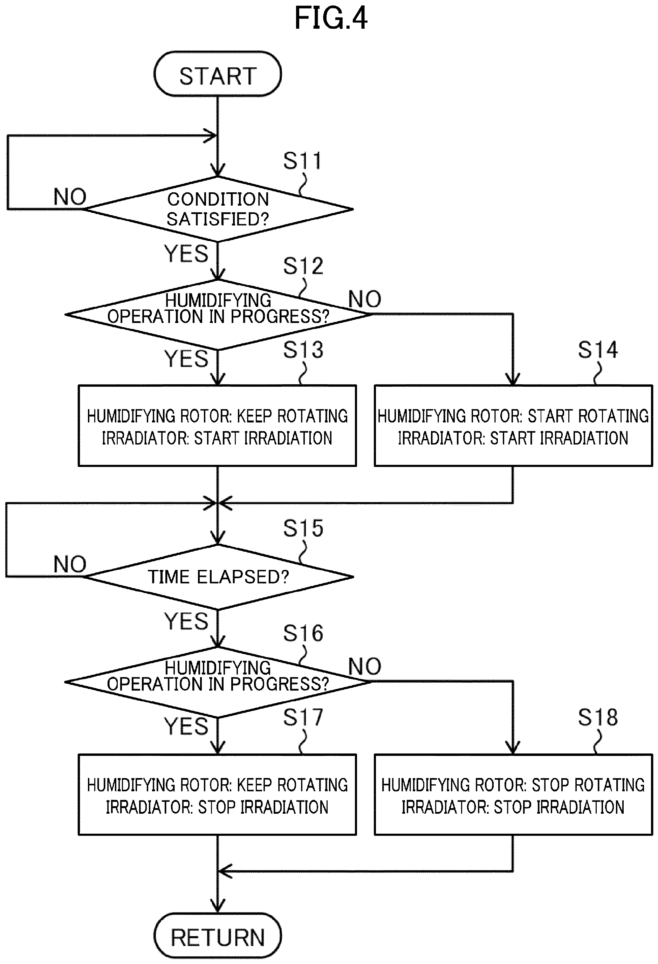

Next, the first control will be described with reference to FIG. 4. The controller (73) repeatedly performs the following process.

### <Step (S11)>

First, the controller (73) determines whether or not a condition for starting the first control is satisfied. If the condition for starting the first control is satisfied, the process goes to Step (S12).

In this example, the controller (73) performs the first control every time a predetermined time elapses. The condition for starting the first control is the condition that the predetermined time has elapsed from the time when the previous first control was ended (or from the time when the humidifier (10) was powered on. The same applies hereinafter). Specifically, when the previous first control is ended, the controller (73) starts measuring an elapsed time. Then, the controller (73) determines whether or not the predetermined time has elapsed from the time when the previous first control was ended. If the predetermined time has elapsed from the time when the previous first control was ended, the process goes to Step (S12).

### <Step (S12)>

If the condition for starting the first control is satisfied, the controller (73) determines whether or not the humidifying operation is in progress in the humidifier (10). For example, the controller (73) determines that the humidifying operation is in progress if the humidifying rotor (30) and the fan (50) are in the driving mode, and determines that the humidifying operation is not in progress if the humidifying rotor (30) and the fan (50) are in the stop mode.

If the humidifying operation is in progress, the process goes to Step (S13). If not, the process goes to Step (S14).

### <Step (S13)>

If the humidifying operation is in progress in the humidifier (10), the controller (73) controls the humidifying rotor (30) and the irradiator (40) so that the humidifying rotor (30) keeps rotating and the irradiator (40) starts emitting ultraviolet light.

When the irradiator (40) starts emitting ultraviolet light, the controller (73) starts measuring the time of ultraviolet irradiation by the irradiator (40) (elapsed time from the start of irradiation). Next, the process goes to Step (S15).

### <Step (S14)>

If the humidifying operation is not in progress in the humidifier (10), the controller (73) controls the humidifying rotor (30) and the irradiator (40) so that the humidifying rotor (30) starts rotating and the irradiator (40) starts emitting ultraviolet light.

When the irradiator (40) starts emitting ultraviolet light, the controller (73) starts measuring the time of ultraviolet irradiation by the irradiator (40) (elapsed time from the start of irradiation). Next, the process goes to Step (S15).

### <Step (S15)>

The controller (73) determines whether or not the time of ultraviolet irradiation by the irradiator (40) has reached a target irradiation time set in advance. When the time of ultraviolet irradiation by the irradiator (40) reaches the target irradiation time, the process goes to Step (S16). For example, the target irradiation time is set to a time that enables the ultraviolet irradiation by the irradiator (40) to sufficiently sterilize the microorganisms adhering to the humidifying rotor (30).

### <Step (S16)>

When the time of ultraviolet irradiation by the irradiator (40) reaches the target irradiation time, the controller (73) determines whether or not the humidifying operation is in progress in the humidifier (10). If the humidifying operation is in progress, the process goes to Step (S17). If not, the process goes to Step (S 18).

### <Step (S17)>

If the humidifying operation is in progress in the humidifier (10), the controller (73) controls the humidifying rotor (30) and the irradiator (40) so that the humidifying rotor (30) keeps rotating and the irradiator (40) stops emitting ultraviolet light. Next, the process goes to Step (S11).

### <Step (S18)>

If the humidifying operation is not in progress in the humidifier (10), the controller (73) controls the humidifying rotor (30) and the irradiator (40) so that the humidifying rotor (30) stops rotating and the irradiator (40) stops emitting ultraviolet light. Next, the process goes to Step (S 11).

### [Advantages of Embodiment]

As described above, the humidifier (10) of the embodiment includes the humidifying rotor (30) that rotates so that the portion of the humidifying rotor (30) having absorbed moisture in the water storage tank (24) moves to the air passage (23) and releases the moisture, and also includes the irradiator (40) that irradiates part of the humidifying rotor (30) with ultraviolet light.

With the above configuration, the humidifying rotor (30) can be irradiated with ultraviolet light, and the microorganisms adhering to the humidifying rotor (30) can be sterilized or inactivated. Thus, microbial growth in the humidifying rotor (30) can be reduced.

The first control can be performed to control the humidifying rotor (30) and the irradiator (40) so that the irradiator (40) emits ultraviolet light when the humidifying rotor (30) is rotated. Thus, part of the humidifying rotor (30) can be irradiated with ultraviolet light while the humidifying rotor (30) is rotated. Thus, a wide area of the humidifying rotor (30) can be irradiated with ultraviolet light. For example, since the entirety of the humidifying rotor (30) can be irradiated with ultraviolet light, the entirety of the humidifying rotor (30) can be effectively sterilized.

In addition, part of the humidifying rotor (30) is irradiated with ultraviolet light while the humidifying rotor (30) is rotated, and thus a wide area of the humidifying rotor (30) can be irradiated with ultraviolet light. Thus, the output of the irradiator (40) can be reduced in comparison with the situation where a wide area of the humidifying rotor (30) is irradiated with ultraviolet light without the humidifying rotor (30) being rotated.

Further, part of the humidifying rotor (30) is irradiated with ultraviolet light while the humidifying rotor (30) is rotated, and thus unevenness in ultraviolet irradiation of the humidifying rotor (30) can be reduced in comparison with the case where a wide area of the humidifying rotor (30) is irradiated with ultraviolet light without the humidifying rotor (30) being rotated. Thus, the humidifying rotor (30) can be efficiently sterilized with ultraviolet light.

In the humidifier (10) of the embodiment, the irradiator (40) irradiates part of the portion of the humidifying rotor (30) positioned in the air passage (23) with ultraviolet light.

With the above configuration, the humidifying rotor (30) can be irradiated with ultraviolet light without irradiation of the portion of the humidifying rotor (30) positioned in the water storage tank (24). Note that since ultraviolet light is easily absorbed by water, it is difficult for ultraviolet light to reach the portion of the humidifying rotor (30) positioned in the water storage tank (24).

Thus, the humidifying rotor (30) is irradiated with ultraviolet light without irradiation of the portion of the humidifying rotor (30) positioned in the water storage tank (24), thereby enabling the irradiator (40) to efficiently irradiate the humidifying rotor (30) with ultraviolet light. Thus, the output of the irradiator (40) can be reduced in comparison with the case where the portion of the humidifying rotor (30) positioned in the water storage tank (24) is irradiated with ultraviolet light.

In the humidifier (10) of the embodiment, if the first control is started when the humidifying operation is in progress, the controller (73) controls the humidifying rotor (30) and the irradiator (40) so that the humidifying rotor (30) keeps rotating and the irradiator (40) starts emitting ultraviolet light.

With the above configuration, the first control can be performed when the humidifying operation is in progress. Thus, the humidifying rotor (30) can be sterilized with ultraviolet light while the humidifying operation is continued.

In the humidifier (10) of the embodiment, if the first control is started when the humidifying operation is not in progress, the controller (73) controls the humidifying rotor (30) and the irradiator (40) so that the humidifying rotor (30) starts rotating and the irradiator (40) starts emitting ultraviolet light.

With the above configuration, the first control can be performed when the humidifying operation is not in progress. Thus, the humidifying rotor (30) can be sterilized with ultraviolet light when the humidifying operation is stopped.

In the humidifier (10) of the embodiment, the controller (73) performs the first control every time the predetermined time elapses.

With the above configuration, the first control can be automatically performed every predetermined time. Thus, the first control can be periodically performed.

In the humidifier (10) of the embodiment, since microbial growth in the humidifying rotor (30) can be reduced, generation of slime (biofilm), generation of odor due to microbial volatile organic compounds (MVOC), resuspension of microorganisms grown in the humidifying rotor (30) into air in the air passage (23), etc. can be reduced.

### (First Variation)

A humidifier (10) of a first variation of the embodiment is different from the humidifier (10) of the embodiment in the setting of the number of rotations of the humidifying rotor (30) in the first control. Other configurations of the humidifier (10) of the first variation of the embodiment are the same as those of the humidifier (10) of the embodiment.

In the first variation, the number of rotations of the humidifying rotor (30) is variable. Specifically, the number of rotations of the humidifying rotor (30) can be switched in multiple levels (two or more levels).

In the first variation, the number of rotations of the humidifying rotor (30) in the first control performed when the humidifying operation is not in progress is less than the number of rotations of the humidifying rotor (30) in the first control performed when the humidifying operation is in progress.

Specifically, in the first variation, if the first control is started when the humidifying operation is in progress (i.e., if Step (S13)), the controller (73) controls the humidifying rotor (30) so that the number of rotations of the humidifying rotor (30) is a first number of rotations. If the first control is started when the humidifying operation is not in progress (i.e., if Step (S 14)), the controller (73) controls the humidifying rotor (30) so that the number of rotations of the humidifying rotor (30) is a second number of rotations that is less than the first number of rotations.

The first number of rotations may be the number of rotations of the humidifying rotor (30) in the humidifying operation where the first control is not performed (the target number of rotations set in normal humidifying operation).

As described above, in the humidifier (10) of the first variation of the embodiment, the number of rotations of the humidifying rotor (30) in the first control performed when the humidifying operation is not in progress is less than the number of rotations of the humidifying rotor (30) in the first control performed when the humidifying operation is in progress.

With the above setting, when the humidifying operation is not in progress, the amount of ultraviolet irradiation (product of the illuminance and the irradiation time) of the humidifying rotor (30) during one rotation of the humidifying rotor (30) can be increased. Thus, when the humidifying operation is not in progress, it is possible to improve a sterilization effect (effect of sterilizing the humidifying rotor (30) with ultraviolet light) during one rotation of the humidifying rotor (30).

Since the sterilization effect (effect of sterilizing the humidifying rotor (30) with ultraviolet light) during one rotation of the humidifying rotor (30) is improved, it is possible to reduce movement of microorganisms adhering to the humidifying rotor (30) in the air passage (23) to the water storage tank (24) without the microorganisms being sterilized.

For example, when the humidifying operation is not in progress, the amount of ultraviolet irradiation of the humidifying rotor (30) during one rotation of the humidifying rotor (30) can be set to the "amount of ultraviolet irradiation which can sufficiently sterilize microorganisms adhering to the humidifying rotor (30)." Thus, the humidifying rotor (30) can be sufficiently sterilized during one rotation of the humidifying rotor (30). This can prevent microorganisms adhering to the humidifying rotor (30) in the air passage (23) from moving to the water storage tank (24) without being sterilized (contamination of the water storage tank (24)). In addition, it is possible to prevent the humidifying rotor (30) sterilized with ultraviolet light from coming into contact with water stored in the water storage tank (24) and being contaminated again.

### (Second Variation)

A humidifier (10) of a second variation of the embodiment is different from the humidifier (10) of the embodiment in the setting of a predetermined time that serves as a condition for starting the first control. Other configurations of the humidifier (10) of the second variation of the embodiment are the same as those of the humidifier (10) of the embodiment.

In the second variation, the predetermined time is settable. The operating person of the humidifier (10) can change the predetermined time to any time. Specifically, when the operator (71) is given operation for changing the predetermined time, the controller (73) changes, in response to such operation, the predetermined time (predetermined time used in Step (S11)) that serves as the condition for starting the first control. The predetermined time may be stored in the storage (72).

As described above, in the humidifier (10) of the second variation of the embodiment, the predetermined time is settable. With the above setting, the cycle of the first control can be changed. For example, the operating person of the humidifier (10) can set the cycle of the first control to any cycle by setting the predetermined time to any time.

### (Third Variation)

A humidifier (10) of a third variation of the embodiment is different from the humidifier (10) of the embodiment in timing of executing the first control. Other configurations of the humidifier (10) of the third variation of the embodiment are the same as those of the humidifier (10) of the embodiment.

There is a correlation between the "environmental condition around the humidifier (10)" and the "ease of microbial growth (growth rate) in the humidifying rotor (30)." Hereinafter, the environmental condition around the humidifier (10) will be simply referred to as an "environmental condition," and the ease of microbial growth (growth rate) in the humidifying rotor (30) will be simply referred to as a "microbial growth rate." For each type of microorganism, there is an environmental condition (for example, optimum temperature and humidity) where such a microorganism is likely to grow. For example, as the environmental condition goes into high temperature and high humidity, the microbial growth rate is faster. Preferably, as the microbial growth rate is faster, the frequency of execution of the first control is greater.

In the third variation, the controller (73) controls the timing of executing the first control according to the environmental condition around the humidifier (10). For example, the controller (73) controls the timing of executing the first control according to the environmental condition so that the frequency of execution of the first control is greater as the environmental condition goes into high temperature and high humidity.

Specifically, the controller (73) sets the predetermined time (predetermined time used in Step (S11)) that serves as the condition for starting the first control according to a temperature-humidity condition indicating the "temperature around the humidifier (10)" detected by the temperature sensor (61) and the "humidity around the humidifier (10)" detected by the humidity sensor (62). For example, as the temperature-humidity condition goes into high temperature and high humidity, the predetermined time is shorter.

As described above, in the humidifier (10) of the third variation of the embodiment, the controller (73) controls the timing of executing the first control according to the environmental condition around the humidifier (10). With such control, the timing of executing the first control can be controlled according to the environmental condition around the humidifier (10), which is correlated with the ease of microbial growth in the humidifying rotor (30). Thus, microbial growth in the humidifying rotor (30) can be reduced properly.

In the third variation, information (correspondence table) indicating the correspondence between the temperature-humidity condition and the predetermined time may be stored in the storage (72). The controller (73) may refer to the information stored in the storage (72) to set the predetermined time according to the temperature-humidity condition.

In the third variation, the controller (73) may start the first control as follows.

The controller (73) measures the time elapsed after the end of the previous first control (or after the humidifier (10) is powered on). Hereinafter, the time elapsed after the end of the previous first control (or after the humidifier (10) is powered on) will be referred to as a "non-sterilization time." Further, the controller (73) derives the "microbial growth rate" according to the environmental condition around the humidifier (10).

Next, the controller (73) derives the "level of microbial growth in the humidifying rotor (30)" according to the product of the "non-sterilization time" and the "microbial growth rate." Hereinafter, the level of microbial growth in the humidifying rotor (30) will be simply referred to as a "microbial growth level." For example, as the product of the non-sterilization time and the microbial growth rate is larger, the microbial growth level is higher.

The controller (73) starts the first control when the microbial growth level exceeds an allowable value set in advance.

As described above, in the third variation, the condition for starting the first control may be the condition that the microbial growth level exceeds the allowable value.

### (Fourth Variation)

A humidifier (10) of a fourth variation of the embodiment is different from the humidifier (10) of the embodiment in control of the irradiator (40). Other configurations of the humidifier (10) of the fourth variation of the embodiment are the same as those of the humidifier (10) of the embodiment.

As described above, there is the correlation between the "environmental condition" and the "microbial growth rate." In addition, as the product of the "microbial growth rate" and the "non-sterilization time (time elapsed after the end of the previous first control)" is larger, the "microbial growth level" is higher. Preferably, as the microbial growth level is higher, a "target irradiation amount," which is a target value of the amount of ultraviolet irradiation by the irradiator (40) in the first control, is larger.

The target irradiation amount is the product of a "target illuminance" which is a target value of the illuminance of ultraviolet light from the irradiator (40) in the first control and the "target irradiation time" which is a target value of the time of ultraviolet irradiation by the irradiator (40) in the first control. For example, the target irradiation amount is set to an ultraviolet irradiation amount which enables sufficient sterilization of microorganisms adhering to the humidifying rotor (30).

In the fourth variation, the illuminance or irradiation time of ultraviolet light from the irradiator (40) is variable. The controller (73) controls the illuminance or irradiation time of ultraviolet light from the irradiator (40) in the first control according to the environmental condition around the humidifier (10). For example, the controller (73) controls the illuminance or irradiation time of ultraviolet light from the irradiator (40) in the first control according to the environmental condition around the humidifier (10) so that the ultraviolet irradiation amount which is the product of the illuminance and irradiation time of ultraviolet light from the irradiator (40) is larger as the environmental condition goes into high temperature and high humidity.

Specifically, the controller (73) derives the "microbial growth rate" according to the temperature-humidity condition indicating the "temperature around the humidifier (10)" detected by the temperature sensor (61) and the "humidity around the humidifier (10)" detected by the humidity sensor (62). For example, as the temperature-humidity condition goes into high temperature and high humidity, the microbial growth rate is faster. Next, the controller (73) derives the "target irradiation amount" according to the product of the "microbial growth rate" and the "non-sterilization time." For example, as the product of the microbial growth rate and the non-sterilization time is larger, the target irradiation amount is larger. Next, the controller (73) derives the "target illuminance" and the "target irradiation time" from the target irradiation amount. Then, in the first control, the controller (73) controls the irradiator (40) so that the illuminance and irradiation time of ultraviolet light from the irradiator (40) reach the target illuminance and the target irradiation time.

The above "illuminance or irradiation time of ultraviolet light from the irradiator (40)" means "at least one of illuminance and irradiation time of ultraviolet light from the irradiator (40)." The same applies hereinafter.

As described above, in the humidifier (10) of the fourth variation of the embodiment, the controller (73) controls the illuminance or irradiation time of ultraviolet light from the irradiator (40) in the first control according to the environmental condition around the humidifier (10). With such control, the amount of ultraviolet irradiation by the irradiator (40) (product of the illuminance and the irradiation time) in the first control can be controlled according to the environmental condition around the humidifier (10), which is correlated with the ease of microbial growth in the humidifying rotor (30). Thus, microbial growth in the humidifying rotor (30) can be reduced properly.

If the predetermined time (predetermined time used in Step (S11)) that serves as the condition for starting the first control is constant, the non-sterilization time is constant. In this case, the controller (73) can unambiguously derive the target irradiation amount according to the environmental condition around the humidifier (10). For example, as the environmental condition goes into high temperature and high humidity, the target irradiation amount is larger.

If the target irradiation time is constant, the time of ultraviolet irradiation by the irradiator (40) in the first control is constant. If the non-sterilization time is constant and the target irradiation time is constant, the controller (73) can unambiguously derive the target illuminance according to the environmental condition around the humidifier (10). For example, as the environmental condition goes into high temperature and high humidity, the target illuminance is stronger.

If the target illuminance is constant, the illuminance of ultraviolet light from the irradiator (40) in the first control is constant. If the non-sterilization time is constant and the target illuminance is constant, the controller (73) can unambiguously derive the target irradiation time according to the environmental condition around the humidifier (10). For example, as the environmental condition goes into high temperature and high humidity, the target irradiation time is longer.

### (Fifth Variation)

A humidifier (10) of a fifth variation of the embodiment is different from the humidifier (10) of the embodiment in timing of executing the first control. Other configurations of the humidifier (10) of the fifth variation of the embodiment are the same as those of the humidifier (10) of the embodiment.

For each type of microorganism, it is possible to predict the ease of microbial growth (growth rate). Preferably, as the microbial growth rate is faster, the frequency of execution of the first control is greater.

In the fifth variation, the controller (73) controls the timing of executing the first control according to the specified type of microorganism. For example, for each type of microorganism, the microbial growth rate (for example, growth rate under an environmental condition set in advance) is set in advance. The controller (73) controls the timing of executing the first control according to the specified type of microorganism so that the frequency of execution of the first control is greater as the specified microbial growth rate is faster.

Specifically, the operating person of the humidifier (10) gives the operator (71) operation for specifying the type of microorganism as a sterilization target. For example, the type of microorganism as the sterilization target is the type of microorganism assumed to be present in the environment around the humidifier (10). When the operator (71) is given the operation for specifying the type of microorganism, the controller (73) sets the predetermined time (predetermined time used in Step (S11)) that serves as the condition for starting the first control according to the "microbial growth rate" corresponding to the specified type of microorganism. For example, as the specified microbial growth rate is faster, the predetermined time is shorter.

As described above, in the humidifier (10) of the fifth variation of the embodiment, the controller (73) controls the timing of executing the first control according to the specified type of microorganism. With such control, the timing of executing the first control can be controlled according to the ease of microbial growth corresponding to the specified type of microorganism. Thus, microbial growth in the humidifying rotor (30) can be reduced properly.

In the fifth variation, information (correspondence table) indicating the correspondence between the type of microorganism and the predetermined time may be stored in the storage (72). The controller (73) may refer to the information stored in the storage (72) to set the predetermined time according to the specified type of microorganism.

In the fifth variation, the controller (73) may start the first control as follows.

The controller (73) measures the "non-sterilization time." The non-sterilization time is the time elapsed after the end of the previous first control (or after the humidifier (10) is powered on). Further, the controller (73) derives the "microbial growth rate" according to the specified type of microorganism.

Next, the controller (73) derives the "microbial growth level" according to the product of the "non-sterilization time" and the "microbial growth rate." For example, as the product of the non-sterilization time and the microbial growth rate is larger, the microbial growth level is higher.

The controller (73) starts the first control when the microbial growth level exceeds the allowable value set in advance.

As described above, in the fifth variation, the condition for starting the first control may be the condition that the microbial growth level exceeds the allowable value.

### (Sixth Variation)

A humidifier (10) of a sixth variation of the embodiment is different from the humidifier (10) of the embodiment in control of the irradiator (40). Other configurations of the humidifier (10) of the sixth variation of the embodiment are the same as those of the humidifier (10) of the embodiment.

As described above, for each type of microorganism, it is possible to predict the ease of microbial growth (growth rate). In addition, as the product of the "microbial growth rate" and the "non-sterilization time (time elapsed after the end of the previous first control)" is larger, the "microbial growth level" is higher. Preferably, as the growth level is higher, the target irradiation amount (product of the target illuminance and the target irradiation time) is larger.

In the sixth variation, the illuminance or irradiation time of ultraviolet light from the irradiator (40) is variable. The controller (73) controls the illuminance or irradiation time of ultraviolet light from the irradiator (40) in the first control according to the specified type of microorganism. For example, when the operator (71) is given operation for specifying the type of microorganism, the controller (73) controls the illuminance or irradiation time of ultraviolet light from the irradiator (40) in the first control according to the specified type of microorganism so that the amount of ultraviolet irradiation (product of the illuminance and the irradiation time) by the irradiator (40) in the first control is larger as the "microbial growth rate" corresponding to the specified type of microorganism is faster.

Specifically, the controller (73) derives the "microbial growth rate" according to the specified type of microorganism. Next, the controller (73) derives the "target irradiation amount" according to the product of the "microbial growth rate" and the "non-sterilization time." For example, as the product of the microbial growth rate and the non-sterilization time is larger, the target irradiation amount is larger. Next, the controller (73) derives the "target illuminance" and the "target irradiation time" from the target irradiation amount. Then, in the first control, the controller (73) controls the irradiator (40) so that the illuminance and irradiation time of ultraviolet light from the irradiator (40) reach the target illuminance and the target irradiation time.

As described above, in the humidifier (10) of the sixth variation of the embodiment, the controller (73) controls the illuminance or irradiation time of ultraviolet light from the irradiator (40) in the first control according to the specified type of microorganism. With such control, the amount of ultraviolet irradiation (product of the illuminance and the irradiation time) by the irradiator (40) in the first control can be controlled according to the ease of microbial growth corresponding to the specified type of microorganism. Thus, microbial growth in the humidifying rotor (30) can be reduced properly.

If the predetermined time (predetermined time used in Step (S11)) that serves as the condition for starting the first control is constant, the non-sterilization time is constant. In this case, the controller (73) can unambiguously derive the target irradiation amount according to the specified type of microorganism. For example, as the "microbial growth rate" corresponding to the specified type of microorganism is faster, the target irradiation amount is larger.

If the target irradiation time is constant, the time of ultraviolet irradiation by the irradiator (40) in the first control is constant. If the non-sterilization time is constant and the target irradiation time is constant, the controller (73) can unambiguously derive the target illuminance according to the specified type of microorganism. For example, as the "microbial growth rate" corresponding to the specified type of microorganism is faster, the target illuminance is stronger.

If the target illuminance is constant, the illuminance of ultraviolet light from the irradiator (40) in the first control is constant. If the non-sterilization time is constant and the target illuminance is constant, the controller (73) can unambiguously derive the target irradiation time according to the specified type of microorganism. For example, as the "microbial growth rate" corresponding to the specified type of microorganism is faster, the target irradiation time is longer.

### (Seventh Variation)

A humidifier (10) of a seventh variation of the embodiment is different from the humidifier (10) of the embodiment in timing of executing the first control. Other configurations of the humidifier (10) of the seventh variation of the embodiment are the same as those of the humidifier (10) of the embodiment.

There is a correlation between the "cumulative operation time of the humidifying operation in the humidifier (10)" and the "microbial growth level in the humidifying rotor (30)." Hereinafter, the cumulative operation time of the humidifying operation in the humidifier (10) will be simply referred to as a "cumulative operation time." For example, as the cumulative operation time is longer, the microbial growth level is higher. Preferably, as the microbial growth level is higher, the frequency of execution of the first control is greater.

In the seventh variation, the controller (73) measures the cumulative operation time. Specifically, the controller (73) starts measuring the cumulative operation time when the humidifying operation is started, and keeps measuring the cumulative operation time when the humidifying operation is in progress and interrupts measurement of the cumulative operation time when the humidifying operation is not in progress. When the first control is started, the controller (73) resets the cumulative operation time to zero.

In the seventh variation, the controller (73) controls the timing of executing the first control according to the cumulative operation time of the humidifying operation in the humidifier (10). For example, the controller (73) controls the timing of executing the first control according to the cumulative operation time so that the frequency of execution of the first control is greater as the cumulative operation time is longer.

Specifically, the controller (73) sets the predetermined time (predetermined time used in Step (S11)) that serves as the condition for starting the first control according to the cumulative operation time. For example, as the cumulative operation time is longer, the predetermined time is shorter.

As described above, in the humidifier (10) of the seventh variation of the embodiment, the controller (73) controls the timing of executing the first control according to the cumulative operation time of the humidifying operation in the humidifier (10). With such control, the timing of executing the first control can be controlled according to the cumulative operation time of the humidifying operation which is correlated with the microbial growth level in the humidifying rotor (30). Thus, microbial growth in the humidifying rotor (30) can be reduced properly.

In the seventh variation, information (correspondence table) indicating the correspondence between the cumulative operation time and the predetermined time may be stored in the storage (72). The controller (73) may refer to the information stored in the storage (72) to set the predetermined time according to the cumulative operation time.

In the seventh variation, the controller (73) may start the first control as follows.

The controller (73) derives the "microbial growth level" according to the "cumulative operation time." For example, as the cumulative operation time is longer, the microbial growth level is higher. The controller (73) starts the first control when the microbial growth level exceeds the allowable value set in advance.

As described above, in the seventh variation, the condition for starting the first control may be the condition that the microbial growth level exceeds the allowable value.

### (Eighth Variation)

A humidifier (10) of an eighth variation of the embodiment is different from the humidifier (10) of the embodiment in control of the irradiator (40). Other configurations of the humidifier (10) of the eighth variation of the embodiment are the same as those of the humidifier (10) of the embodiment.

As described above, there is the correlation between the "cumulative operation time" and the "microbial growth level." Preferably, as the growth level is higher, the target irradiation amount (product of the target illuminance and the target irradiation time) is larger.

In the eighth variation, the illuminance or irradiation time of ultraviolet light from the irradiator (40) is variable. The controller (73) controls the illuminance or irradiation time of ultraviolet light from the irradiator (40) in the first control according to the cumulative operation time of the humidifying operation in the humidifier (10). For example, the controller (73) controls the illuminance or irradiation time of ultraviolet light from the irradiator (40) in the first control according to the cumulative operation time so that the amount of ultraviolet irradiation (product of the illuminance and the irradiation time) by the irradiator (40) in the first control is larger as the cumulative operation time is longer.

Specifically, the controller (73) derives the "target irradiation amount" according to the cumulative operation time. For example, as the cumulative time is longer, the target irradiation amount is larger. Next, the controller (73) derives the "target illuminance" and the "target irradiation time" from the target irradiation amount. Then, in the first control, the controller (73) controls the irradiator (40) so that the illuminance and irradiation time of ultraviolet light from the irradiator (40) reach the target illuminance and the target irradiation time.

As described above, in the humidifier (10) of the eighth variation of the embodiment, the controller (73) controls the illuminance or irradiation time of ultraviolet light from the irradiator (40) in the first control according to the cumulative operation time of the humidifying operation in the humidifier (10). With such control, the amount of ultraviolet irradiation (product of the illuminance and the irradiation time) by the irradiator (40) in the first control can be controlled according to the cumulative operation time of the humidifying operation which is correlated with the microbial growth level in the humidifying rotor (30). Thus, microbial growth in the humidifying rotor (30) can be reduced properly.

If the target irradiation time is constant, the time of ultraviolet irradiation by the irradiator (40) in the first control is constant. In this case, the controller (73) can unambiguously derive the target illuminance according to the cumulative operation time of the humidifying operation in the humidifier (10). For example, as the cumulative operation time is longer, the target illuminance is stronger.

If the target illuminance is constant, the illuminance of ultraviolet light from the irradiator (40) in the first control is constant. In this case, the controller (73) can unambiguously derive the target irradiation time according to the cumulative operation time of the humidifying operation in the humidifier (10). For example, as the cumulative operation time is longer, the target irradiation time is longer.

### (Ninth Variation)

A humidifier (10) of a ninth variation of the embodiment is different from the humidifier (10) of the embodiment in control of the humidifying rotor (30) and control of the irradiator (40). Other configurations of the humidifier (10) of the ninth variation of the embodiment are the same as those of the humidifier (10) of the embodiment.

In the ninth variation, the number of rotations of the humidifying rotor (30) is variable. Specifically, the number of rotations of the humidifying rotor (30) can be switched in multiple levels (two or more levels).

In the ninth variation, the illuminance of ultraviolet light from the irradiator (40) is variable. In the first control, the controller (73) controls the illuminance of ultraviolet light from the irradiator (40) according to the number of rotations of the humidifying rotor (30). For example, in the first control, the controller (73) controls the illuminance of ultraviolet light from the irradiator (40) according to the number of rotations of the humidifying rotor (30) so that the illuminance of ultraviolet light from the irradiator (40) is stronger as the number of rotations of the humidifying rotor (30) is larger.

Specifically, in the first control, the controller (73) derives the target illuminance according to the number of rotations of the humidifying rotor (30). For example, as the number of rotations of the humidifying rotor (30) is larger, the target illuminance is stronger. Then, the controller (73) controls the illuminance of ultraviolet light from the irradiator (40) so that the illuminance of ultraviolet light from the irradiator (40) reaches the target illuminance.

In the ninth variation, the controller (73) controls the number of rotations of the humidifying rotor (30) according to a humidification load in the humidifying operation. For example, in the humidifying operation, the controller (73) controls the number of rotations of the humidifying rotor (30) according to the humidification load so that the number of rotations of the humidifying rotor (30) is larger as the humidification load is heavier. As the number of rotations of the humidifying rotor (30) is larger, humidification of air in the air passage (23) by the humidifying rotor (30) is more promoted.

Specifically, in the humidifying operation, the controller (73) derives the "humidification load" by subtracting the "humidity of air around the humidifier (10)" from the "target humidity" set in advance. Next, the controller (73) derives a "target number of rotations" according to the humidification load. For example, as the humidification load is heavier, the target number of rotations is larger. The controller (73) controls the number of rotations of the humidifying rotor (30) so that the number of rotations of the humidifying rotor (30) reaches the target number of rotations.

As described above, in the humidifier (10) of the ninth variation of the embodiment, in the first control, the controller (73) controls the illuminance of ultraviolet light from the irradiator (40) according to the number of rotations of the humidifying rotor (30). With such control, it is possible to properly control the amount of ultraviolet irradiation (product of the illuminance and the irradiation time) of the humidifying rotor (30).

For example, the amount of ultraviolet irradiation (product of the illuminance and the irradiation time) of the humidifying rotor (30) during one rotation of the humidifying rotor (30) can be maintained at a predetermined amount. Thus, it is possible to sufficiently ensure the sterilization effect (effect of sterilizing the humidifying rotor (30) with ultraviolet light) during one rotation of the humidifying rotor (30). As a result, it is possible to reduce movement of microorganisms adhering to the humidifying rotor (30) in the air passage (23) to the water storage tank (24) without the microorganisms being sterilized.

In the humidifier (10) of the ninth variation of the embodiment, the controller (73) controls the number of rotations of the humidifying rotor (30) according to the humidification load in the humidifying operation. With such control, the humidifying rotor (30) can promote humidification of air in the air passage (23) according to the humidification load, and thus humidification can be properly performed.

In the ninth variation, in the first control, the controller (73) may control the illuminance of ultraviolet light from the irradiator (40) according to the number of rotations of the humidifying rotor (30) so that the product of a "time required for one rotation of the humidifying rotor (30)" derived from the number of rotations of the humidifying rotor (30) and the "illuminance of ultraviolet light from the irradiator (40)" reaches the target irradiation amount set in advance. With such control, the amount of ultraviolet irradiation (product of the illuminance and the irradiation time) of the humidifying rotor (30) during one rotation of the humidifying rotor (30) can be maintained at the target irradiation amount.

For example, if the target irradiation amount is set to the "ultraviolet irradiation amount which enables sufficient sterilization of microorganisms adhering to the humidifying rotor (30)," the humidifying rotor (30) can be sufficiently sterilized during one rotation of the humidifying rotor (30). This can prevent microorganisms adhering to the humidifying rotor (30) in the air passage (23) from moving to the water storage tank (24) without being sterilized (i.e., it can prevent contamination of the water storage tank (24)). In addition, it is possible to prevent the humidifying rotor (30) sterilized with ultraviolet light from coming into contact with water stored in the water storage tank (24) and then from being contaminated again.

### (Other Embodiments)

In the above descriptions, the air purifier having the humidifying function and the air purifying function has been described as an example of the humidifier (10), but the humidifier (10) is not limited thereto. For example, the humidifier (10) may be a humidifier having only a humidifying function.

While the embodiments and variations thereof have been described above, it will be understood that various changes in form and details may be made without departing from the spirit and scope of the claims. The foregoing embodiments and variations thereof may be combined and replaced with each other without deteriorating the intended functions of the present disclosure.

### INDUSTRIAL APPLICABILITY

As described above, the present disclosure is useful as a humidifier.

### DESCRIPTION OF REFERENCE CHARACTERS

- 10: Humidifier
- 20: Housing
- 21: Suction Port
- 22: Blow-Out Port
- 23: Air Passage
- 24: Water Storage Tank
- 30: Humidifying Rotor
- 35: Driver
- 40: Irradiator
- 50: Fan
- 61: Temperature Sensor
- 62: Humidity Sensor
- 63: Rotation Number Sensor
- 64: Illuminance Sensor
- 71: Operator
- 72: Storage
- 73: Controller

## Claims

1. A humidifier comprising:
a housing (20) formed with an air passage (23) through which air passes and a water storage tank (24) configured to store water;
a humidifying rotor (30) extending over the air passage (23) and the water storage tank (24) and configured to rotate so that a portion of humidifying rotor (30) having absorbed moisture from the water storage tank (24) moves to the air passage (23) and releases the moisture; and
an irradiator (40) configured to irradiate part of the humidifying rotor (30) with ultraviolet light.

2. The humidifier of claim 1, further comprising:
a controller (73) configured to perform first control,
wherein
in the first control, the controller (73) controls the humidifying rotor (30) and the irradiator (40) so that the irradiator (40) emits ultraviolet light when the humidifying rotor (30) is rotated.

3. The humidifier of claim 2, wherein
if the first control is started when humidifying operation where the humidifying rotor (30) is rotated is in progress, the controller (73) controls the humidifying rotor (30) and the irradiator (40) so that the humidifying rotor (30) keeps rotating and the irradiator (40) starts emitting ultraviolet light.

4. The humidifier of claim 2 or 3, wherein
if the first control is started when the humidifying operation where the humidifying rotor (30) is rotated is not in progress, the controller (73) controls the humidifying rotor (30) and the irradiator (40) so that the humidifying rotor (30) starts rotating and the irradiator (40) starts emitting ultraviolet light.

5. The humidifier of claim 4, wherein
the number of rotations of the humidifying rotor (30) in the first control performed when the humidifying operation is not in progress is less than the number of rotations of the humidifying rotor (30) in the first control performed when the humidifying operation is in progress.

6. The humidifier of any one of claims 2 to 5, wherein
the controller (73) performs the first control every time a predetermined time elapses.

7. The humidifier of claim 6, wherein
the predetermined time is settable.

8. The humidifier of any one of claims 2 to 7, wherein
the controller (73) controls timing of executing the first control according to an environmental condition around the humidifier.

9. The humidifier of any one of claims 2 to 8, wherein
the controller (73) controls an illuminance or irradiation time of ultraviolet light from the irradiator (40) in the first control according to the environmental condition around the humidifier.

10. The humidifier of any one of claims 2 to 9, wherein
the number of rotations of the humidifying rotor (30) is variable, and
in the first control, the controller (73) controls the illuminance of ultraviolet light from the irradiator (40) according to the number of rotations of the humidifying rotor (30).

11. The humidifier of claim 10, wherein
the controller (73) controls the number of rotations of the humidifying rotor (30) according to a humidification load in the humidifying operation where the humidifying rotor (30) is rotated.
